Europäisches Patentamt

European Patent Office

Office européen des brevets

(19)

(11) Numéro de publication : **0 519 829 A1**

# (12) DEMANDE DE BREVET EUROPEEN

(21) Numéro de dépôt : **92401711.4**

(22) Date de dépôt : **19.06.92**

(51) Int. Cl.⁵ : **C12N 15/81**

(30) Priorité : **21.06.91 FR 9107640**

(43) Date de publication de la demande :
**23.12.92 Bulletin 92/52**

(84) Etats contractants désignés :
**PT**

(71) Demandeur : **RHONE-POULENC RORER SA**
**20, avenue Raymond Aron**
**F-92160 Antony (FR)**

(72) Inventeur : **Chen, Xin-Jie Research School**
**Biological Sciences**
**Australia National University GPO Box 475**
**AU-Camberra ACT 2601 (AU)**
Inventeur : **Fleer, Reinhard**
**47 avenue Beauséjour**
**F-91440 Bures sur Yvette (FR)**
Inventeur : **Fukuhara, Hiroshi, 160 avenue du**
**Général Leclerc**
**Résidence de Courcelles, Bât. 7**
**F-91190 Gif Sur Yvette (FR)**

(74) Mandataire : **Savina, Jacques et al**
**Rhône-Poulenc Rorer S.A. Direction Brevets**
**(t144) 20 avenue Raymond Aron**
**F-92165 Antony Cédex (FR)**

(54) **Vecteurs de clonage et/ou d'expression préparation et utilisation.**

(57) L'invention concerne un nouveau plasmide de levure, de nouveaux vecteurs de clonage et/ou d'expression dérivés de celui-ci et leur utilisation.

FIGURE 1

EP 0 519 829 A1

La présente invention concerne un nouveau plasmide de levure, ainsi que de nouveaux vecteurs de clonage et/ou d'expression dérivés de celui-ci, leur préparation et leur utilisation, notamment pour la production de protéines recombinantes. L'invention concerne aussi les cellules hôtes recombinées contenant de tels vecteurs.

Plus particulièrement, la présente invention concerne tout ou partie d'un nouveau plasmide isolé à partir de la levure Kluyveromyces waltii ou d'un dérivé de celui-ci, et les vecteurs de clonage et/ou d'expression construits à partir de celui-ci.

Au cours des dix dernières années, la levure est apparue comme un microorganisme hôte très prometteur pour la production de protéines hétérologues.

En particulier, la mise en évidence par Beggs et al (Nature 275 (1978) 104) du plasmide 2μ et de vecteurs dérivés de celui-ci a été l'une des clés du développement actuel de l'étude génétique et moléculaire de la levure Saccharomyces cerevisiae. Depuis, le système 2μ a permis d'introduire et d'exprimer des gènes hétérologues dans la levure afin d'obtenir des protéines d'intérêt pharmaceutique ou agro-alimentaire. Cependant, le plasmide 2μ et ses dérivés ne peuvent se répliquer efficacement que dans les levures appartenant à l'espèce S.cerevisiae et à quelques espèces proches de celle-ci. Ce système ne peut donc pas être utilisé pour la manipulation génétique dans la plupart des espèces de levure. Notamment, il n'est pas utilisable dans les levures dont les propriétés physiologiques, distinctes de celles de S.cerevisiae, permettraient des applications nouvelles et/ou des exploitations industrielles plus performantes.

Différents laboratoires ont donc recherché d'autres plasmides dans des espèces variées de levure. Ainsi, plusieurs plasmides circulaires ont été trouvés chez la levure du genre Zygosaccharomyces, et notamment les plasmides pSR1 et pSR2 (Toh-é et al., J. Bacteriol. 151 (1982) 1380) ; pSB1, pSB2, pSB3 et pSB4 (Toh-é et al., J. Gen. Microbiol., 130 (1984) 2527) ; et pSM1 (Utatsu et al., J. Bacteriol. 169 (1987) 5537). Un plasmide circulaire a également été trouvé chez Kluyveromyces drosophilarum: pKD1 (Falcone et al., Plasmid 15 (1986) 248).

Tous ces plasmides présentent des caractéristiques communes avec le plasmide 2μ, et notamment des séquences répétées inversées, et l'existence sous deux formes isomériques possibles due à un système de recombinaison site-spécifique.

Cependant, ces plasmides présentent toujours l'inconvénient d'avoir un spectre d'hôte étroit. Ainsi, en raison de leur spécificité vis-à-vis des espèces hôtes de levure, ces plasmides ne peuvent être utilisés que dans un nombre limité de souches.

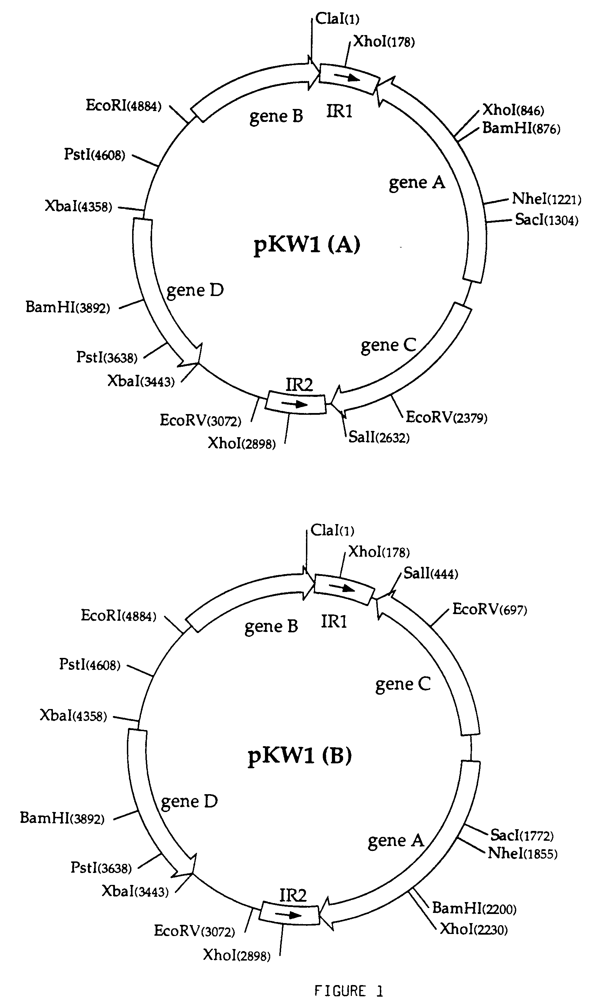

La présente invention résulte de la mise en évidence d'un plasmide naturel de la levure Kluyveromyces waltii. Ce plasmide, nommé pKW1, est le premier plasmide naturel connu de cette espèce de levure. Purifié à partir de la souche K.waltii CBS 6430, ce plasmide a été cartographié au moyen d'enzymes de restrictions, et la carte résultante est présentée sur la figure 1.

Un objet de l'invention réside donc dans le plasmide pKW1 isolé à partir de la souche K.waltii CBS6430, ou tout fragment ou dérivé de celui-ci.

Par dérivé, on entend au sens de l'invention les plasmides qui, en dépit de quelques modifications, conservent les propriétés du plasmide de départ. Notamment, les modifications peuvent prendre la forme de mutations ou de délétions portant sur des régions d'une importance relative. Il peut s'agir également d'insertions ou de suppressions, comme par exemple de sites de clonage.

Par fragment de pKW1, on entend notamment les différents éléments génétiques de ce plasmide. Plus préférentiellement, comme éléments génétiques de pKW1, on peut citer notamment les gènes de structure ou des parties de ceux-ci, des séquences promotrices fonctionnelles, les séquences répétées inversées (IR), ou encore les séquences permettant la réplication (origine de réplication) ou conférant la stabilité au plasmide (locus de stabilité).

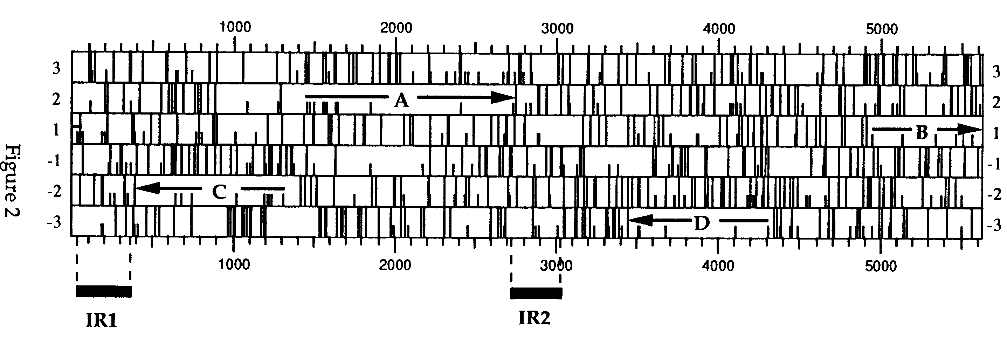

En effet, l'étude structurale du plasmide pKW1 a permis de mettre en évidence des analogies avec le plasmide 2μ de S.cerevisiae. Ainsi, 4 gènes de structure ont été mis en évidence (voir figure 2), ainsi qu'une origine de réplication. Par ailleurs, le clonage de pKW1 dans E. coli a permis d'isoler 4 types de plasmides recombinés, correspondant à 2 formes isomériques du plasmide pKW1 (les formes A et B) clonées dans le vecteur pKan21 dans les deux orientations possibles (le vecteur pKan21 est décrit dans l'exemple 3.1). Ces deux formes sont représentées sur la figure 1. L'existence de deux formes isomériques indique la présence de séquences répétées inversées. L'étude a ainsi montré que le plasmide pKW1 contient une paire de séquences répétées inversées de 0,3 kb chacune, et deux séquences uniques de 2,5 et 2,3 kb, dont l'orientation distingue les 2 formes isomériques A et B. La taille moléculaire du plasmide pKW1 est donc de 5,5 kb environ. Différents sites de restriction ont été mis en évidence et, à titre d'exemple, les sites uniques suivants : EcoRI, SphI, SalI, ClaI, NheI et BglI.

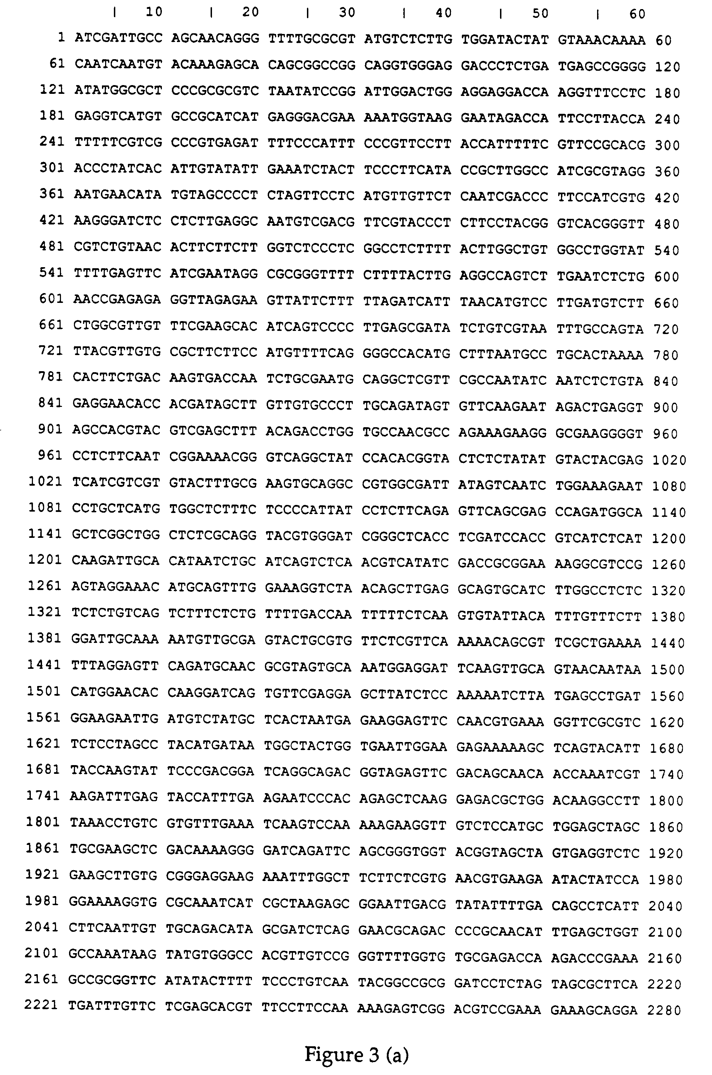

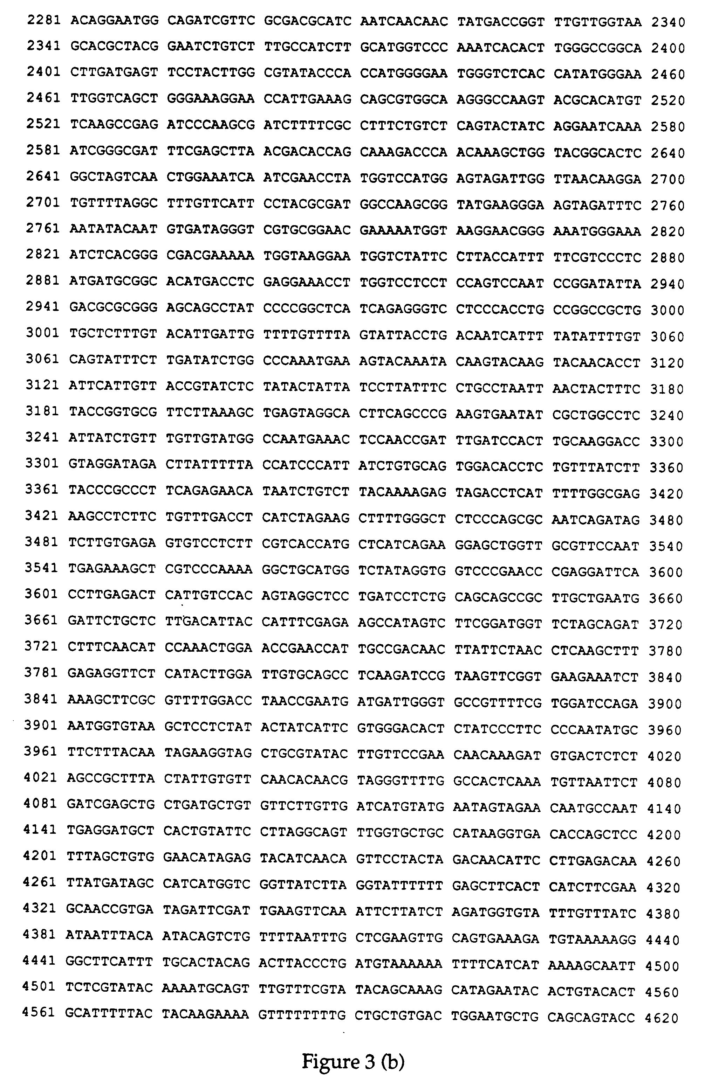

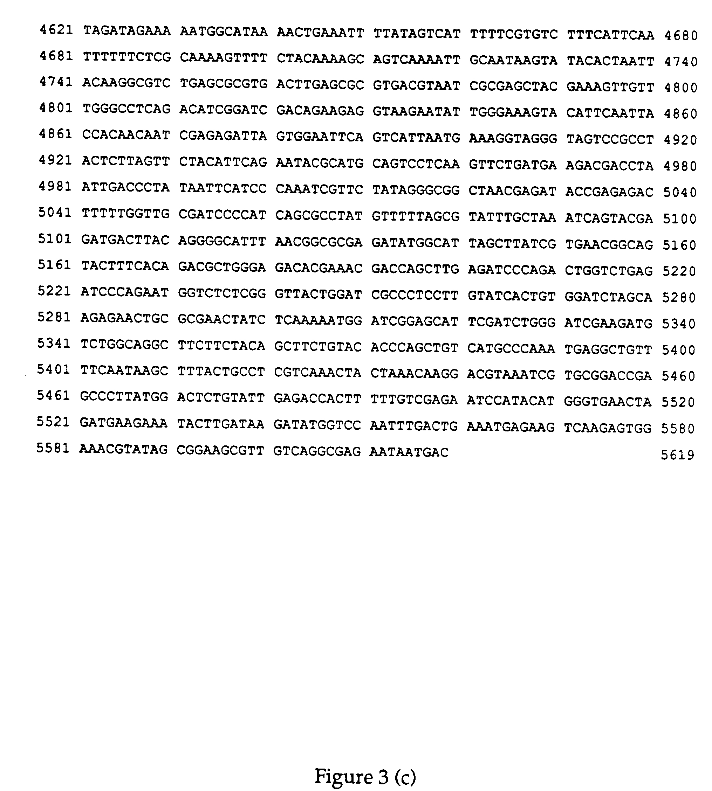

La séquence nucléotidique complète du plasmide pKW1 a également été déterminée (figure 3). L'absence d'homologie entre cette séquence et celle de plasmides connus a été démontrée, notamment par des expériences d'hybridation (voir exemple 2). L'absence d'hybridation en conditions de stringence modérée est caractéristique de cette différence de séquence.

Dans un mode préféré, l'invention a pour objet un plasmide comprenant tout ou partie de la séquence présentée à la figure 3 ou d'un dérivé de celle-ci.

Par ailleurs, la Demanderesse a également montré qu'il est possible d'utiliser le plasmide pKW1 ou des fragments de celui-ci pour construire des vecteurs de clonage et/ou d'expression particulièrement stables.

Un autre objet de la présente invention réside donc dans des vecteurs de clonage et/ou d'expression caractérisés en ce qu'ils comprennent tout ou partie du plasmide pKW1 de K.waltii CBS 6430 représenté à la figure 1, ou d'un dérivé de celui-ci.

Un objet plus particulier de l'invention consiste en un vecteur de clonage et/ou d'expression caractérisé en ce qu'il comprend un élément génétique au moins du plasmide pKW1.

En raison du spectre d'hôte du plasmide pKW1, les vecteurs de l'invention peuvent être utilisés dans des espèces autres que l'hôte naturel K. waltii.

Ils peuvent notamment être utilisés pour la transformation d'espèces très variées, notamment d'espèces de levures.

Différents types de vecteurs ont été construits à partir de pKW1, différents au niveau de la taille du fragment provenant de pKW1, et donc des éléments fonctionnels issus de pKW1.

Un objet encore plus particulier de l'invention consiste en un vecteur de clonage et/ou d'expression caractérisé en ce qu'il comprend l'origine de réplication du plasmide pKW1.

D'autres constructions peuvent être préparées, contenant des fragments plus ou moins importants, permettant d'étudier l'influence des différents éléments de pKW1 sur la stabilité des vecteurs, leur spécificité d'hôte, et leur efficacité pour l'expression de gènes hétérologues. En particulier, des vecteurs d'expression peuvent être élaborés à partir des différents éléments génétiques du plasmide pKW1 (origine de réplication, séquences répétées inversées, gènes de structuré, régions promotrices ..), que l'on peut introduire dans des plasmides connus pour améliorer leurs performances ou leur conférer de nouvelles propriétés. De la même façon, des vecteurs peuvent être obtenus en ajoutant des éléments au plasmide pKW1, ou en remplaçant certains éléments génétiques de pKW1 par des éléments provenant d'autres plasmides. Ainsi, des vecteurs peuvent être obtenus en substituant par exemple l'origine de réplication de pKW1 par l'origine de réplication du plasmide 2μ de S. cerevisiae ou du plasmide pKD1 de Kluyveromyces, ou par un réplicon chromosomique (ARS) d'une levure (exemple KARS de K.lactis). De la même façon, des vecteurs peuvent être obtenus en substituant le locus de stabilité de pKW1 par celui du plasmide 2μ de S. cerevisiae ou du plasmide pKD1 de Kluyveromyces. Il peut être particulièrement intéressant de réaliser des vecteurs hybrides comprenant des éléments des plasmides pKD1 et pKW1.

Avantageusement, les vecteurs de l'invention comprennent l'intégralité du plasmide pKW1 tel que représenté à la figure 1.

Préférentiellement, les vecteurs de l'invention comprennent le plasmide pKW1 linéarisé à un site de restriction fonctionellement neutre.

Par site de restriction fonctionellement neutre, on entend au sens de la présente invention un site de restriction au niveau duquel il est possible d'interrompre la structure du plasmide sans altérer ses propriétés de réplication et de stabilité.

En particulier, il peut s'agir de sites présents sur le plasmide pKW1. A titre d'exemple, on peut citer notamment les sites ClaI(1); PstI(4608); ou EcoRV(3072) tels que représentés sur la figure 1.

Il peut également s'agir de sites introduits artificiellement sur le plasmide pKW1, ou rendus uniques. Dans ce cas, les sites sont préférentiellement introduits dans des régions intergéniques du plasmide, et notamment dans la région située entre les gènes B et D ou dans celle située entre le gène D et l'IR2.

Avantageusement, selon la présente invention, le plasmide pKW1 est linéarisé au niveau d'un site de restriction unique.

Un site particulièrement interessant à cet égard est le site unique ClaI localisé en position 1 sur la figure 1. La Demanderesse a en effet montré que ce site permettait d'utiliser le plasmide pKW1 pour construire les vecteurs de clonage et/ou d'expression, en introduisant à ce niveau, par exemple, des fragments d'ADN hétérologue, tout en maintenant la réplication stable du vecteur obtenu. Ce résultat est surprenant dans la mesure où le site ClaI est localisé dans le gène de structure B.

L'utilisation de tels sites de clonage neutres permet donc d'obtenir des vecteurs très stables, capables de se maintenir dans les cellules transformées, même en l'absence de toute pression de sélection.

Avantageusement, les vecteurs de l'invention contiennent en outre une séquence d'ADN hétérologue comprenant un gène de structure au moins, sous le contrôle de signaux permettant son expression.

Les signaux permettant l'expression du ou des gènes de structure peuvent être constitués par un ou plusieurs éléments choisis parmi les promoteurs, les terminateurs ou les signaux de sécrétion. Il est entendu que ces signaux sont choisis en fonction de l'hôte utilisé, du gène de structure et du résultat recherché. En particulier il peut être préférable dans certains cas d'utiliser un promoteur régulable, permettant le découplage entre les phases de croissance de l'hôte et d'expression du ou desdits gènes de structure. De même, l'utilisation d'un peptide signal (signal de sécrétion) peut permettre d'augmenter les taux de production de la protéine recherchée, et faciliter l'étape de purification.

Préférentiellement, les promoteurs utilisés sont dérivés de gènes de levure. D'un intérêt tout particulier sont les promoteurs dérivés de gènes glycolytiques des levures du genre Saccharomyces ou Kluyveromyces. Notamment, on peut citer les promoteurs des gènes codant pour la phosphoglycerate kinase de S.cerevisiae (PGK), la Glyceraldehyde-3-phosphate deshydrogenase (GPD), les énolases (ENO), ou les alcool-déshydrogenases (ADH). On peut également citer des promoteurs dérivés de gènes fortement exprimés tels que le gène de la lactase (LAC4) ou de la phosphatase acide (PHO5).

Par ailleurs, ces régions peuvent être modifiées par mutagénèse, par exemple pour ajouter des éléments supplémentaires de contrôle de la transcription, tels que notamment des régions UAS ("Upstream Activating Sequence").

Le gène de structure qui peut être introduit dans les vecteurs de l'invention code préférentiellement pour un polypeptide d'intérêt pharmaceutique ou agroalimentaire. A titre d'exemple, on peut citer les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur van Willebrand, la fibronectine, l'alpha-1 antitrypsine etc.), l'insuline et ses variants, les lymphokines [telles que les interleukines, les interférons, les facteurs de stimulation des colonies (G-CSF, GM-CSF, M-CSF...), le TNF, le TRF etc.], les facteurs de croissance (tels que l'hormone de croissance, l'érythropoïétine, le FGF, l'EGF, le PDGF, le TGF etc.), les apolipoprotéines, ou encore des polypeptides antigéniques pour la réalisation de vaccins (hépatite, cytomégalovirus, Eppstein-Barr, herpes etc.).

Dans un mode particulier de réalisation de l'invention le gène de structure peut être un gène résultant de la fusion de plusieurs séquences d'ADN. Il peut s'agir notamment d'un gène codant pour un polypeptide hybride, contenant par exemple une partie active associée à une partie stabilisatrice. A titre d'exemple, on peut citer les fusions entre l'albumine ou des fragments d'albumine et un récepteur ou une partie d'un récepteur de virus (CD4, etc.).

Dans un autre mode de réalisation, la séquence d'ADN hétérologue peut comprendre plusieurs gènes de structure, et notamment des gènes intervenant, au niveau génétique ou biochimique, dans la biosynthèse d'un métabolite. Le métabolite peut en particulier être un antibiotique, un acide aminé ou une vitamine.

Dans un mode particulier de réalisation, les vecteurs de l'invention contiennent en outre :
- un réplicon E.coli et/ou
- au moins un marqueur de sélection.

Ces éléments permettent de manipuler de manière beaucoup plus aisée les vecteurs de l'invention.

Un autre objet de l'invention concerne les cellules recombinées contenant un vecteur tel que défini ci-avant.

Les cellules recombinées sont préférentiellement choisies parmi les levures.

La Demanderesse a montré que les vecteurs de l'invention pouvaient en effet être utilisés aussi bien dans K.waltii (l'hôte naturel de pKW1) que dans des levures d'espèces ou même de genres différents. Notamment, ils peuvent être utilisés dans d'autres Klyuveromyces ou dans Saccharomyces. Par ailleurs, lorsque la souche K. waltii CBS6430 est utilisée comme cellule hôte, des recombinaisons homologues entre les vecteurs de l'invention et le plasmide pKW1 résident peuvent affecter la stabilité des vecteurs, et ainsi diminuer les performances du couple hôte/vecteur. Afin d'améliorer encore la stabilité des vecteurs de l'invention dans un tel couple hôte/vecteur, la demanderesse a préparé une souche K. waltii pKW1⁻ (KW18). Cette souche permet d'optimiser l'utilisation industrielle des vecteurs de l'invention (voir exemple 4).

Différentes techniques peuvent être utilisées pour introduire les vecteurs de l'invention dans les cellules hôtes. En particulier, la transformation (Bianchi et al., Curr. Genet. 12 (1978) 185) et l'électroporation (Delorme, Appl. Environ. Microbiol. 155 (1989) 2242) donnent de bons résultats. Il est clair toutefois que l'invention n'est pas limitée à une technique particulière.

Un objet de l'invention réside également dans un procédé de préparation d'un polypeptide selon lequel on cultive une cellule recombinée telle que définie plus haut et on récupère le polypeptide produit. Plus particulièrement, le procédé de l'invention permet la production de protéines d'intérêt pharmaceutique ou agroalimentaire, telles que celles indiquées ci-avant. Plus spécifiquement, le procédé de l'invention est adapté à la production d'albumine humaine et de ses variants ou précurseurs.

Dans le cas où les gènes de structure interviennent dans la biosynthèse d'un métabolite, les cellules recombinées peuvent également être utilisées directement dans un procédé de bioconversion.

D'autres avantages de la présente invention apparaîtront à la lecture des exemples qui suivent, qui doivent être considérés comme illustratifs et non limitatifs.

## LEGENDE DES FIGURES

Figure 1 : Carte de restriction du plasmide pKW1. Les séquences répétées inversées ainsi que les gènes de structure A-D sont indiqués. Les positions indiquées pour les sites de restriction correspondent au premier nucléotide reconnu par l'enzyme.

Figure 2 : Etude des phases ouvertes du plasmide pKW1. Les éléments génétiques indiqués sont localisés aux positions suivantes par rapport à la séquence présentée figure 3 : Gène A : nucléotides 1454 à 2755 ; Gène B : nucléotides 4948 à 54 ; Gène C : nucléotides 389 à 1309 sur le brin complémentaire ; Gène D : nucléotides 3444 à 4313 sur le brin complémentaire ; IR1 : nucléotides 53 à 368 ; IR2 : nucléotides 2713 à 3028.

Figure 3 : Séquence nucléotidique du plasmide pKW1. La séquence représentée correspond à la forme B du plasmide. La position 1 correspond au premier nucléotide de la séquence reconnue par l'enzyme ClaI. Le plasmide pKW1 a été fragmenté par des enzymes de restriction et les fragments ont été clonés dans pTZ18R (Pharmacia). Les séquences des segments clonés ont été déterminées par la méthode de Sanger sur les 2 brins.

Figure 4: Cartes de restriction des plasmides navettes YIP5 et pKan21. Ap : Gène de résistance à l'ampicilline ; Tc : Gène de résistance à la tétracycline ; Km : Gène de résistance à la kanamycine (G418) ; LacZ : Gène de structure de la β-galactosidase.

Figure 5 : Stratégie de construction des vecteurs pBNA1, pNEA2, pBNB1/A3, pNEB1, pXXY2 et pXXK3. Voir aussi tableau 1.

Figure 6: Carte de restriction du vecteur pXXK3.

Figure 7: Stratégie de construction des vecteurs pKWC11, pKWS1 et pKWS14.

Figure 8 : Etude de la stabilité des vecteurs pKWC11 et pXXK3 dans la souche K.waltii KW18. Dans chaque cas, un clone transformé a été cultivé en milieu YPG non sélectif pendant le nombre de générations indiqué, puis des aliquots de culture ont été étalés sur boites de milieu YPG gélosé avec et sans G418, afin de déterminer le nombre total des cellules et le nombre de cellules résistant à G418. La stabilité correspond au % de cellules résistantes.

Figure 9 : Stratégie de construction du vecteur d'expression pXPHO5. Abréviations : P = promoteur, T = terminateur, ss = signal de sécrétion, CIP = Calf Intestinal Phosphatase, Km = kanamycine, E = EcoRI, H = HindIII, S = SalI, B = BamHI, Sm = SmaI.

Figure 10 : Cartes de restriction des vecteurs pXKN18 et pXPHO5. Légende : voir figure 9.

Figure 11 : Détection immunologique de l'IL1β produite par K.waltii. Les marqueurs de poids moléculaire (KDa) sont indiqués à gauche. Puits 1 : 1L-1β de référence (100 ng) ; Puits 2: Surnageant de culture du transformant pXKN18 (sans cassette IL-1β) ; Puits 3 : Surnageant de culture du transformant pXPHO5 traité par l'endo-N- acetylglucosamidase H ; Puits 4 et 5 : Surnageants des cultures des transformants pXPHO5 en milieu LPi et HPi respectivement.

Figure 12 : Stratégie de construction du plasmide pYG65.

Figure 13 : Stratégie de construction du plasmide pYG70.

Figure 14 : Stratégie de construction du plasmide pYG141. aph : gène codant pour l'aminoglycoside 3'-phosphotransférase, conférant la résistance à la kanamycine; bla: gène codant pour la β-lactamase conférant la résistance à l'ampicilline.

Figure 15 : Stratégie de construction du plasmide pYG142.

Tableaux 1 et 2 : Composition de vecteurs dérivés de pKW1 selon l'invention. (∗) Sites de clonage.

Tableaux 3, 4 et 5 : Transformation par des vecteurs de l'invention respectivement de S.cerevisiae, K.waltii et de différentes souches de Kluyveromyces. La stabilité des transformants est exprimée par le pourcentage de cellules Ura+ après 10 générations de croissance dans le milieu non-sélectif YPD. Les souches isonucléaires K.waltii pKW1+ et pKW1− sont CBS 6430 et KW18 respectivement.

## TECHNIQUES GENERALES DE CLONAGE

Les méthodes classiques de biologie moléculaire telles que la centrifugation d'ADN plasmidique en gradient de chlorure de césium - bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning : a Laboratory Manual", Cold Spring Harbor Laboratory, Cold Spring Harbor, N.Y., 1986; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain Reaction") dans les conditions décrites par Mullis et Faloona (Meth. Enzym., 155 (1987) 335-350) et Saiki et al (Science 230 (1985) 1350-1354), en utilisant un "DNA thermal cycler" (Perkin Elmer Cetus) en suivant les recommandations du fabricant.

EXEMPLES

1) Isolement et purification de pKW1

La souche CBS 6430 est cultivée dans 2 litres de milieu YPG (Yeast extract 1 %, Bactopeptone 1 %, Glucose 2 %) avec agitation à 26°C pendant environ 18 heures. Les cellules en phase stationnaire précoce sont récoltées par centrifugation. On obtient habituellement 13 à 15 grammes de masse cellulaire par litre. Les cellules sont lavées avec 150 ml de sorbitol 1M contenant 30 mg de Zymolyase 20T (Kirin Breweries Co., Tokyo). Après incubation à 30°C pendant 1 heure, on ajoute à la suspension de protoplastes 5 ml de sodium dodecylsulfate à 10 % et 5 ml de EDTA à 0,5M, pH 7,0. Le mélange est immédiatement agité vigoureusement, et incubé à 50°C pendant 1 à 2 heures. On ajoute au lysat l'acétate de potassium à la concentration finale de 1M et le mélange est maintenu dans la glace pendant 2 heures. Les précipités formés sont éliminés par centrifugation (Sorvall SS34, 15 000 tpm, 30 minutes). Le surnageant auquel 2 volumes d'éthanol à 95 % sont ajoutés est refroidi dans la glace afin de précipiter les acides nucléiques. Les précipités sont collectés par centrifugation, lavés avec l'éthanol à 70 %, séchés sous vide, et enfin dissouts dans 40 ml de 5 x TE (1x TE est Tris-HCl 10 mM, EDTA 1mM, pH 8). Après addition de 40 g de CsCl et 5 ml de bromure d'éthidium (solution stock de 10 mg/ml), le mélange est centrifugé à 60 000 tpm pendant 6 heures (Beckman, rotor 60 Ti). La bande fluorescente de l'ADN plasmidique se trouve au-dessous de la bande majeure d'ADN chromosomique. L'ADN du plasmide est collecté et soumis au second cycle de centrifugation dans CsCl/bromure d'éthidium. L'ADN plasmidique collecté est mélangé avec un volume d'isopropanol préalablement équilibré avec CsCl 4M pour éliminer le bromure d'éthidium. Après plusieurs extractions par l'isopropanol, la solution d'ADN est dialysée contre 1 x TE. Le volume de la solution peut être réduit par dialyse contre le polyéthylène-glycol 6 000 en paillettes.

2) Séquençage et études d'homologies

Le plasmide pKW1 a été séquencé en utilisant la méthode décrite par Sanger et al (Proc. Natl. Acad. Sci. USA 74 (1977) 5463-5467). La séquence complète est indiquée sur la figure 3.

L'absence d'homologie entre cette séquence et celle de plasmides connus a été déterminée par des expériences d'hybridations moléculaires : pKW1 est marqué au $^{32}$P, et hybridé avec les plasmides suivants, préalablement immobilisés sur un filtre de nitrocellulose :
- plasmide 2μ de S. cerevisiae,
- pSR1, pSB3 et pSB4 de Z. rouxii,
- pSB1 et pSB2 de Z.bailii, et
- pKD1 de K. drosophilarum.

L'hybridation a été réalisée dans des conditions de stringence modérée (0,6 M Na+, 65°C, 18 heures); aucun des plasmides n'a donné un signal positif d'hybridation.

Les autres plasmides circulaires (pSB1, pSB4) dont les séquences n'ont pas encore été décrites, sont également différents de pKW1, par la taille moléculaire, par la longueur des répétitions inverses et par leurs espèces hôtes.

3) Construction de vecteurs de clonage dérivés de pKW1.

Deux types de molécules recombinées ont été construits à partir de pKW1.

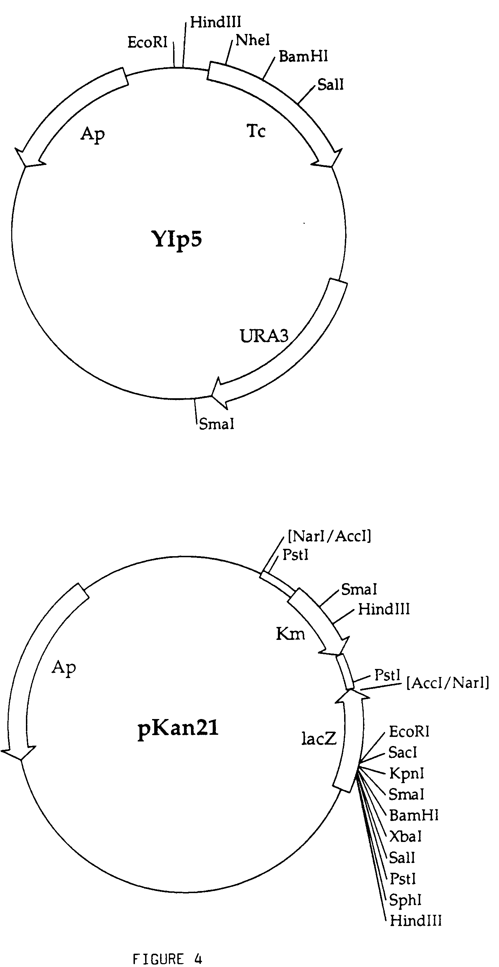

3.1. Dans le premier type, différents fragments de pKW1 (correspondant par exemple aux éléments génétiques de pKW1) ont été introduits dans des vecteurs navette, et notamment dans les vecteurs YIp5 (Struhl et al., Proc. Nat. Acad. Sci USA 76 (1979) 1035) et pKan21, représentés sur la figure 4.

Le plasmide pKan21 a été construit par insertion du gène aph (Genblock, Pharmacia) conférant la résistance à la kanamycine (G418) sous forme d'un fragment AccI de 1,25 kpb, dans le site NarI du plasmide pUC19 (Viera et Messing, Gene 19 (1982) 259). pKan21 contient donc, en plus de aph, le gène bla conférant la résistance à l'ampicilline et l'origine de réplication de ColE1 permettant la réplication dans E. Coli. Les levures transformées par les vecteurs dérivés de pKan21 peuvent être détectées par leur croissance sur milieu contenant 200μg/ml de généticine (G418).

Le plasmide YIp5 est un dérivé du plasmide pBR322 dans lequel le gène URA3 de S.cerevisiae a été inséré comme marqueur de sélection. La levure transformée par les dérivés de YIp5 est détectée par sa croissance sur un milieu sans uracile. Dans ce dernier cas, la levure hôte est une auxotrophe déficiente en orotidine-monophosphate carboxylase de la voie de synthèse de l'uracile.

Les vecteurs de ce premier type sont pBNA1, pNEA2, pBNB1/A3, pNEB1, pXXY2 et pXXK3 (tableau 1 et figure 4).

- Vecteur pBNA1

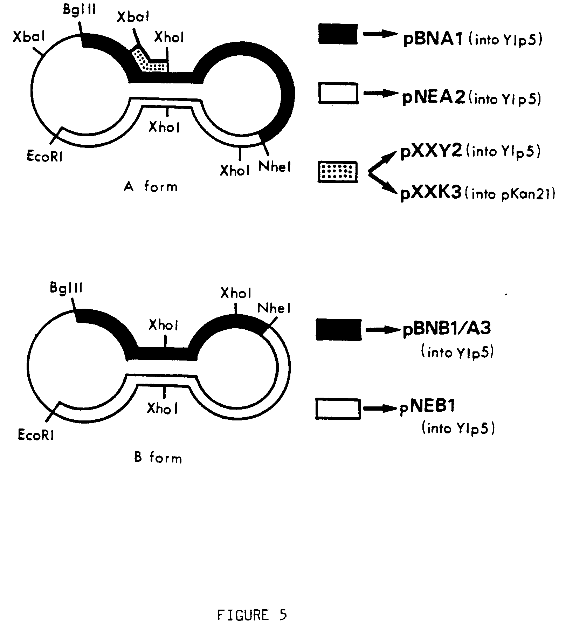

pKW1 a été digéré par BglII et NheI (Cf figure 5) et les fragments d'ADN sont séparés par électrophorèse. Le fragment de 2,4 kpb est récupéré et inséré par ligation entre les sites uniques BamHI et NheI dans le gène de résistance à la tétracycline du plasmide YIp5 (Yanish-Peron et al., Gene 33, 1985, 103-119), celui-ci étant préalablement digéré par BamHI et NheI et repurifié. Les extrémités BglII et BamHI sont compatibles pour ligation spécifique. E. coli transformé par le mélange de ligation est sélectionné sur le milieu LB gélosé contenant l'ampicilline. L'insertion est vérifiée par la réplique des transformants sur le milieu LB gélosé contenant la tétracycline, ceux-ci étant sensibles à cet antibiotique. La structure du plasmide est vérifiée par extraction de l'ADN à partir de transformants individuels et son analyse par les enzymes de restriction. A titre d'exemple, le plasmide pBNA1 digéré par PstI produit 3 fragments de 3,28, 3,15 et 1,36 kpb : la double digestion PstI/NheI donne 4 fragments de 3,15, 2,3, 1,36 et 1,0 kpb.

- Vecteur pNEA2

Le fragment NheI-EcoRI de 2,0 de pKW1 (Cf figure 5) est isolé et inséré entre les deux sites uniques NheI et EcoRI dans le gène de résistance à la tétracycline de YIp5, celui-ci étant préalablement digéré par ces deux enzymes. Le produit de ligation est introduit dans E. coli, et les transformants résistants à l'ampicilline et sensibles à la tétracycline sont isolés. Le plasmide est isolé comme dans le cas de pBNA1 à partir de l'un de ces transformants. La structure du plasmide recombiné obtenu est vérifiée par restriction. A titre d'exemple, la digestion par PstI produit 2 fragments de 4,35 et 3,15 kpb; la double digestion par PstI/XhoI produit 4 fragments de 3,15, 1,8, 1,75 et 0,7 kpb.

- Vecteur pXXY2

Le fragment XhoI-XbaI de 545 pb de pKW1 (Cf figure 5) est isolé et inséré entre les sites uniques SalI-NheI dans le gène de résistance à la tétracycline du plasmide YIp5. Les extrémités XhoI et SalI, d'une part, et XbaI et NheI, d'autre part, sont compatibles pour ligation spécifique. Le produit de ligation est introduit dans E. coli, et le plasmide recombiné est isolé comme dans le cas de pBNA1. La structure du plasmide obtenu est vérifiée par restriction. A titre d'exemple, la digestion par EcoRI + NruI produit deux fragments de 4,5 et 0,96 kpb.

- Vecteur pXXK3

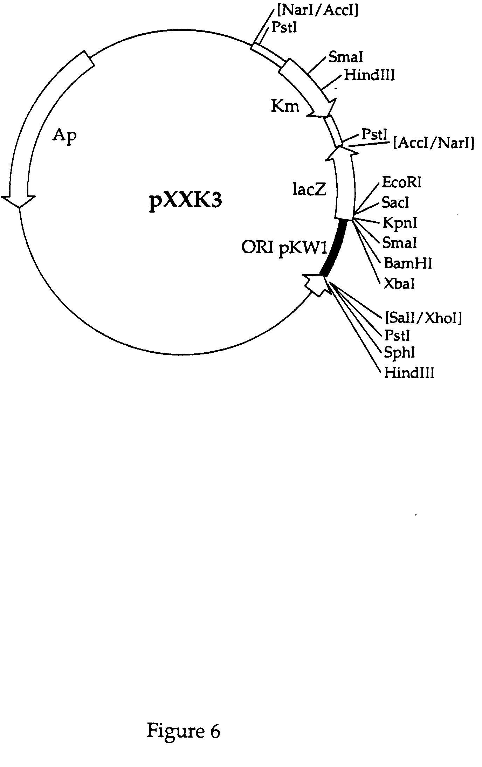

Le fragment XhoI-XbaI de 545 pb de pKW1 (Cf figure 5) est isolé et inséré entre les sites uniques SalI-XbaI (polylinker au N-terminal de LacZ) du plasmide pKan21. Le mélange de ligation est introduit dans E.coli, et les transformants, étalés sur le milieu LB contenant X-gal et IPTG, sont isolés comme colonies blanches parmi les bleues. Répliquées sur le milieu LB contenant la kanamycine, elles croissent bien. Ces colonies sont individuellement analysées pour leur contenu en plasmide. Le plasmide obtenu à partir de l'un des transformants présente la structure montrée dans la figure 6, vérifiée par restriction. A titre d'exemple, la digestion par BamHI + PstI produit quatre fragments 2,5, 1,3, 0,55 et 0,19 kpb.

- Vecteur pBNB1/A3

Le fragment BglII-NheI de 1,9 kpb de pKW1 (Cf figure 5) est isolé et inséré entre les sites BamHI-NheI de YIp5. Le plasmide recombiné est isolé comme dans le cas de pBNA1. La structure du plasmide est vérifiée par restriction. A titre d'exemple, la digestion par PstI produit 3 fragments de 3,15, 2,8 et 1,36 kpb ; la double digestion par PstI/NheI produit 4 fragments de 3,15, 1,8, 1,36 et 1,0 kpb.

- Vecteur pNEB1

Le fragment NheI-EcoRI de 2,5 kpb de pKW1 (Cf figure 5) est isolé et inséré entre les sites NheI-EcoRI de YIp5. Le plasmide recombiné est isolé comme dans le cas de pBNA1. La structure du plasmide est vérifiée par restriction. A titre d'exemple, la digestion par PstI produit 2 fragments de 4,65 et 3,15 kpb ; la double digestion par PstI/XhoI produit 3 fragments de 3,15, 2,9 et 1,75 kpb.

3.2. Le deuxième type de molécules recombinées contient la totalité de la séquence de pKW1. Pour obtenir ces vecteurs, pKW1 est linéalisé par coupure unique à un site de restriction, permettant d'introduire des segments d'ADN hétérologue. De tels segments peuvent contenir des gènes de structure, inclus par exemple dans des cassettes d'expression, et/ou des vecteurs navettes entiers, tels que notamment pKan21 ou YIp5 (figure 4).

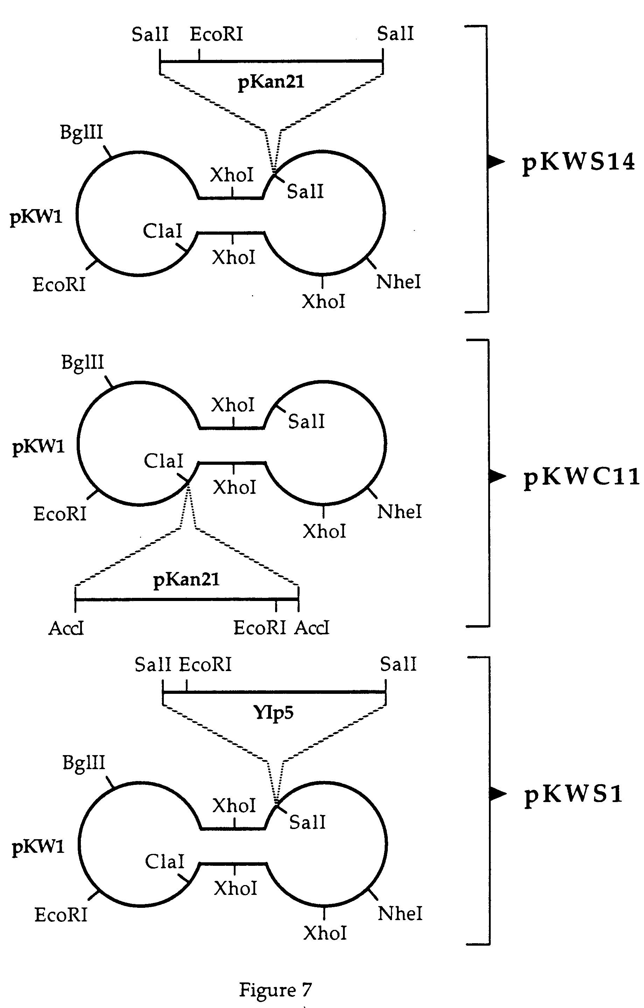

Les exemples de ce type de vecteurs sont pKWC11, pKWS1 et pKWS14 (tableau 2 et figure 7).

- vecteur pKWS14

L'ADN de pKW1 a été digéré par l'enzyme de restriction SalI. Le plasmide pKan21, décrit plus haut, est digéré aussi par SalI (le site unique SalI est localisé dans le multisite de clonage présent dans le gène LacZ). Les deux plasmides sont ligaturés par l'ADN-ligase. Le mélange de ligation est utilisé pour la transformation de E. coli JM83 comme dans le cas précédent. La suspension des cellules transformées est étalée sur LB gélosé contenant X-gal et IPTG. Les colonies blanches, parmi les bleues, sont récupérées individuellement. Elles sont résistantes à l'ampicilline et à la kanamycine. Leur contenu en plasmide est analysé sur les mini-préparations d'ADN comme précédemment. Le plasmide, pKWS14, isolé à partir de l'un des transformants, contient la forme A du plasmide pKW1 et possède la structure montrée dans la Figure 7. Elle est vérifiée par restriction. A titre d'exemple, la digestion par BamHI produit 3 fragments de 5,6, 2,6 et 1,2 kpb.

- Vecteur pKWC11

Le plasmide pKW1 est digéré par ClaI. Le plasmide pKan21 est digéré par AccI. Ils sont repurifiés par traitement au phénol et précipitation par éthanol. Les ADN des deux plasmides sont mélangés en quantités approximativement égales et soumis à la réaction de ligation par l'ADN-ligase pendant une nuit. Le produit de ligation est amplifié dans E. coli. Les colonies des transformants sont blanches sur le milieu LB contenant Xgal et IPTG. Elles sont résistantes à la kanamycine et l'ampicilline sur le milieu contenant l'un ou l'autre de ces antibiotiques. Le plasmide, pKWC11, isolé à partir de l'un des transformants, contient la forme A du plasmide pKW1 et possède la structure montrée dans la Figure 7. Elle est vérifiée par restriction. A titre d'exemple, la digestion du plasmide par BamHI produit 3 fragments de 5,6, 2,9 et 0,9 kpb.

- Vecteur pKWS1

Le plasmide pKW1 et le plasmide YIp5 sont digérés par SalI. Le mélange est repurifié et soumis à la réaction de ligation. Le produit de ligation est introduit dans E. coli. Des transformants résistants à l'ampicilline et sensibles à la tétracycline, sont obtenus. Le plasmide, pKWS1, isolé à partir de l'un d'entre eux, contient la forme A du plasmide pKW1 et possède la structure montrée dans la Figure 7. A titre d'exemple, la digestion du plasmide par EcoRI donne deux fragments de 8,3 et 2,7 kpb.

4) Construction d'une souche CBS 6430 pKW1⁻

K.waltii CBS 6430 a d'abord été transformée par le plasmide recombinant pKWS14 (tableau 2 et figure 7). La méthode de transformation utilisée est essentiellement celle décrite par Chen et Fukuhara (Gene 69, 181 (1988)) utilisant les protoplastes. Les transformants obtenus sont maintenus pendant 75 générations sur milieu YPD-agar 1 mg/ml G418 (Milieu YPD : extrait de levure 10 g/l ; peptone 20 g/l ; glucose 20 g/l). Ils sont ensuite transférés sur milieu YPD liquide sans antibiotique et maintenus pendant 10 générations.

Dans ces conditions (sans pression sélective), le plasmide pKWS14 est progressivement perdu. Les colonies sensibles à G418 qui apparaissent sont prélevées et testées individuellement pour la présence des plasmides. Le test consiste en extraction de l'ADN cellulaire, suivie d'une électrophorèse de ces ADNs sur un gel d'agarose. La présence de plasmides est révélée par coloration au bromure d'éthidium. Parmi les colonies qui sont devenues sensibles à G418, 25 % se sont révélées démunies de tout plasmide. Une de ces colonies a été gardée comme souche de K. waltii dépourvue de plasmide pKW1 et désignée KW18.

### 5) Transformation de différentes levures

### 5.1. Transformation de S.cerevisiae

Parmi les différents vecteurs décrits dans les tableaux 1 et 2 et aux figures 4 et 5, certains de ceux contenant le marqueur URA3 ont été utilisés pour transformer une souche auxotrophe ura3 de S.cerevisiae (la souche S150--2B : Mat a, ura3, leu2, trp1, his3, 2μ).

La méthode de transformation est essentiellement celle décrite par Sherman et al (Yeast Genetics, Cold Spring Harbor, NY, 1986).

Les résultats obtenus sont indiqués dans le tableau 3. Ils montrent que les vecteurs de l'invention sont capables de transformer les levures du genre Saccharomyces.

### 5.2. Transformation de K.waltii

La transformation de K.waltii a été réalisée avec des vecteurs portant le marqueur de résistance à la kanamycine.

La méthode de transformation utilisée est essentiellement celle décrite par Chen et Fukuhara (Gene 69 (1988) 181) utilisant les protoplastes. Il est clair que toute autre technique permettant d'introduire un fragment d'ADN dans un microorganisme peut être utilisée.

Les résultats obtenus sont présentés dans le tableau 4.

Ils montrent que les vecteurs de l'invention sont capables de transformer avec une fréquence élevée la levure K.waltii.

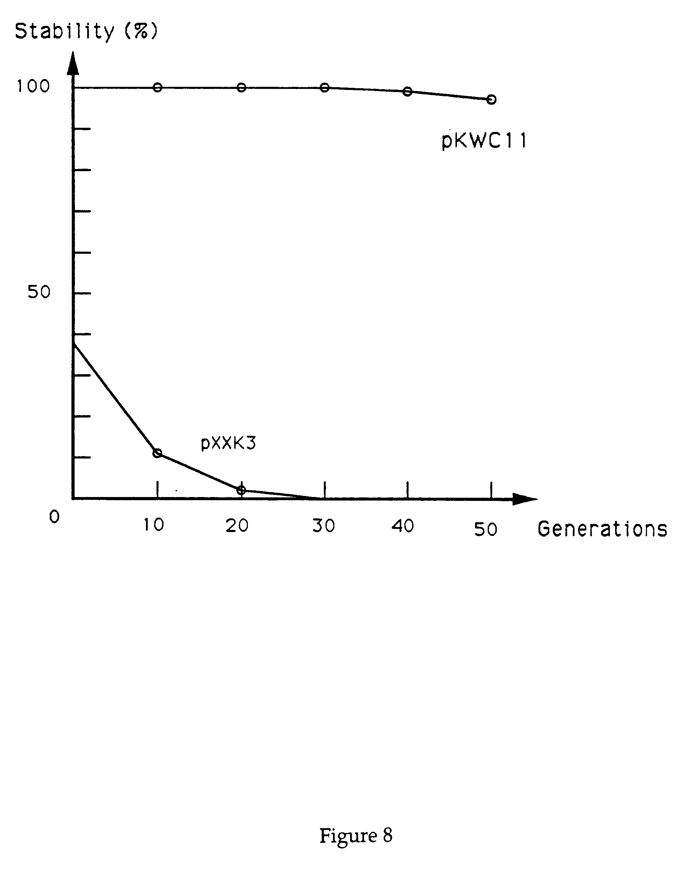

Par ailleurs, l'étude de stabilité décrite dans la figure 8 montre que des vecteurs peuvent être obtenus à partir de pKW1, présentant une stabilité de 100 % après 50 générations de croissance dans un milieu non sélectif. Ceci est parfaitement illustré par le vecteur pKWC11. Cette étude montre également qu'il est préférable, pour obtenir une stabilité relativement élevée, d'utiliser les vecteurs ne comportant que l'origine de réplication du plasmide pKW1 dans des cellules hôtes possédant un plasmide pKW1 résident.

### 5.3. Transformation d'autres levures

Le vecteur pKWC11, hautement stable et autonome dans K.waltii, a été utilisé pour tester la transformabilité de différentes espèces de levures, et notamment celles appartenant au genre Kluyveromyces.

Les résultats sont présentés dans le tableau 5.

La présence du vecteur pKWC11 dans les transformants a été vérifiée par électrophorèse.

L'ensemble de ces résultats montrent que l'étendue des espèces hôtes des vecteurs de l'invention peut être très grande, dépassant le genre Kluyveromyces.

### 6) Utilisation des vecteurs de l'invention pour la production de protéines hétérologues

### 6.1. Interleukine-1β:

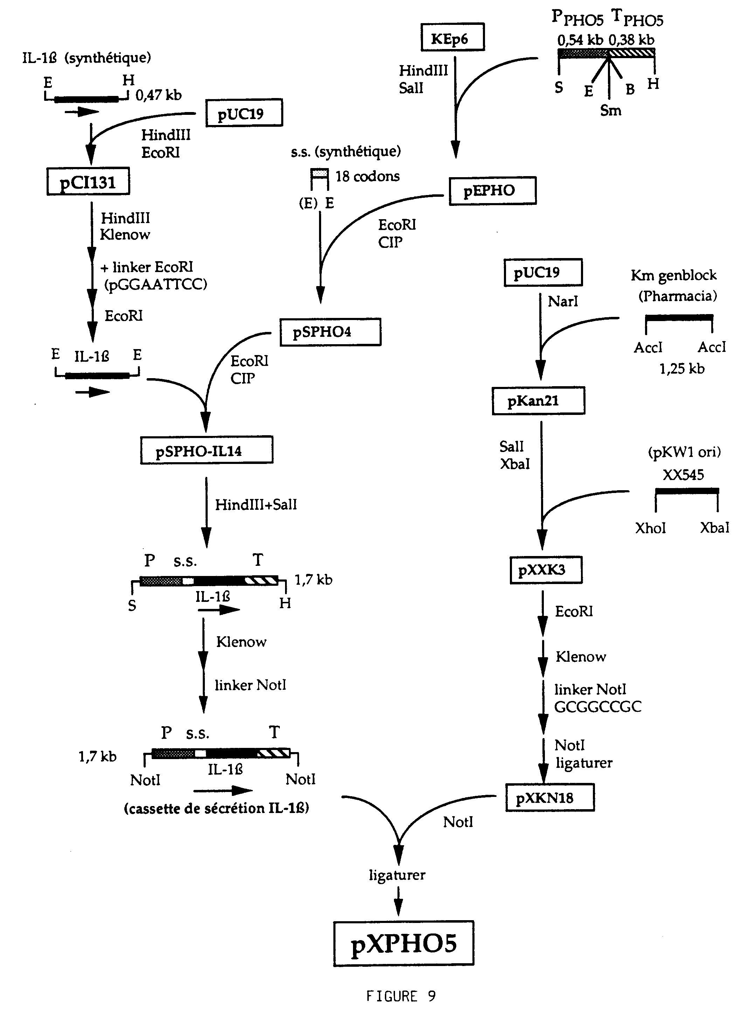

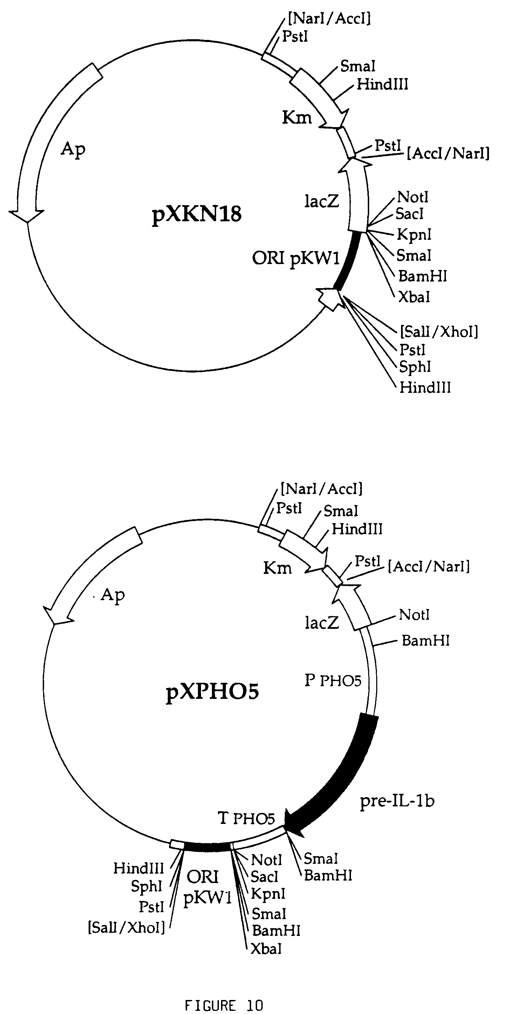

### 6.1.1. Construction d'un vecteur d'expression et de sécrétion de l'IL-1β dérivé de pKW1 (figures 9 et 10).

- Le vecteur pXXK3 (tableau 1, figure 6) est linéarisé par EcoRI et les extrémités sont comblées avec le fragment Klenow de l'ADN polymerase I de E.coli. Un "linker" synthétique (5'-GCGGCCGC-3') formant un site de restriction reconnu par l'enzyme NotI est ajouté au moyen de la ligase T4, et le vecteur ainsi obtenu (pXKN18) est purifié après son amplification chez E.coli (figure 10).

- Une cassette d'expression de l'IL-1β est réalisée, composée (a) du promoteur régulé PHO5 provenant de S. cerevisiae (Bajwa et al., Nucl.Acid.Res. 12, (1984) 7721-7739), (b) du gène de l'IL-1β humaine (Jung et al., Ann. Inst. Pasteur/Microbiol. 139 (1988) 129-146) précédé (c) d'une séquence synthétique correspondant à la séquence signal de la toxine killer de pGKL1 de K.lactis (région pré du gène de la sous-unité alpha) (Stark et Boyd, EMBO J. 5, (1986) 1995-2002), et (d) du terminateur PHO5. La cassette d'expression a été isolée à partir du vecteur pSPHO5-IL14 dont la construction est décrite dans le brevet EP 361991. La cassette a été réalisée de la manière suivante : A l'extrémité 5' du gène codant pour la partie mature de l'IL-1β est insérée la séquence synthétique suivante, sous forme d'un fragment EcoRI :

MetAsnIlePheTyrIlePheLeuPheLeuLeuSerPheValGlnGlyLysArg

5'-AATTATGAATATATTTTACATATTTTTGTTTTTGCTGTCATTCGTTCAAGGTAAAAG-3'

3'-TACTTATATAAAATGTATAAAAACAAAAACGACAGTAAGCAAGTTCCATTTTCTTAA-5'

Les derniers codons ajoutés (Lys et Arg) forment un site de coupure potentiel reconnu par l'endopeptidase Kex1 de K.lactis (Tanguy-Rougeau et al ; FEBS Lett. 234 (1988) 464). Cette séquence a été fusionnée au gène de l'IL-1β par le site EcoRI, formant la jonction suivante :

Gly  Lys  Arg  Ile  His  Met  Ala

5'....GGT AAA AGA ATT CAT ATG GCA ....3'

L'alanine (GCA) correspond au premier acide aminé de l'1L-1β mature. Arg-Ile-His-Met correspond à un "linker" EcoRI-NdeI introduit pour faciliter le clonage (Cf EP 361 991).

L'ensemble de la cassette est mis sous forme d'un fragment NotI par addition d'un linker correspondant (5'-GCGGCCGC-3').

- La cassette de secrétion de l'IL-1β est insérée, au site NotI, dans pXKN18. Le vecteur résultant est appelé pXPHO5 (figure 10).

6.1.2. La souche K.waltii CBS 6430 est transformée par le vecteur pXPHO5, dans les conditions décrites dans l'exemple 5.2.

6.1.3. Expression de l'IL-1β :

Les cellules transformées sont cultivées à 28°C, en absence de G418, dans le milieu liquide LPi (à faible teneur en phosphate minéral) et le milieu HPi (à haute teneur en phosphate), préparés selon Chen et Fukuhara (Gene 69 (1988) 181-192), pendant 4 jours. 50 ml de culture sont centrifugés et les surnageants filtrés sur membrane Millipore (0,22 μm). Les protéines sont précipitées par addition d'éthanol à la concentration finale de 60 %. Les précipités sont dissous dans 2 ml de tampon de Laemmli (Nature 227 (1970) 680-685) et 20 μl d'échantillons sont utilisés pour l'analyse SDS-PAGE selon Laemmli (document précité). Après électrophorèse, les protéines sont transférées sur une feuille de nitrocellulose, et traitées par un antisérum polyclonal de lapin anti IL-1β humaine. Le Blot est ensuite traité avec un 2ème anticorps polyclonal anti-lapin biotinylé (Vectastain ABC ImmunoPeroxydase Kit, Vector Laboratories). Le complexe antigène-anticorps est révélé selon le protocole du fournisseur.

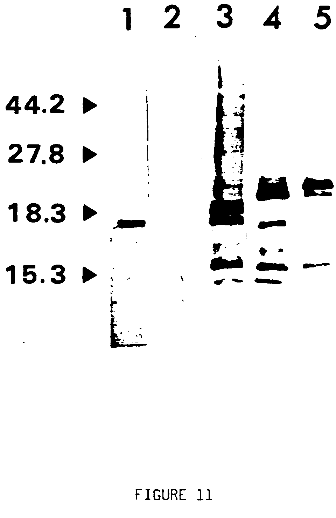

La figure 11 montre qu'une protéine de poids moléculaire apparent de 21 kDa est sécrétée par la levure transformée par pXPHO5. La protéine est spécifiquement reconnue par l'antisérum anti-IL-1β. Cette protéine n'est pas synthétisée par la levure transformée par le vecteur de contrôle pXKN18 (sans cassette 1L-1β). La protéine sécrétée correspond à la forme glycosylé de l'1L-1β, ce qui est démontré par la réduction du PM apparent après traitement par l'enzyme endo-N-acétylglucosamidase H (figure 11 piste 3). Ce couple hôte/vecteur K.waltii/pXPHO5, encore non-optimisé, sécrète approximativement 5 mg d'1L-1βpar litre de culture. Le niveau de la sécrétion d'IL-1β par K.waltii est plus élevé dans le milieu LPi que dans le milieu HPi, ce qui suggère que l'activité du promoteur PHO5 est régulée par le phosphate dans K.waltii comme dans S.cerevisiae.

6.2. Sérum-albumine humaine :

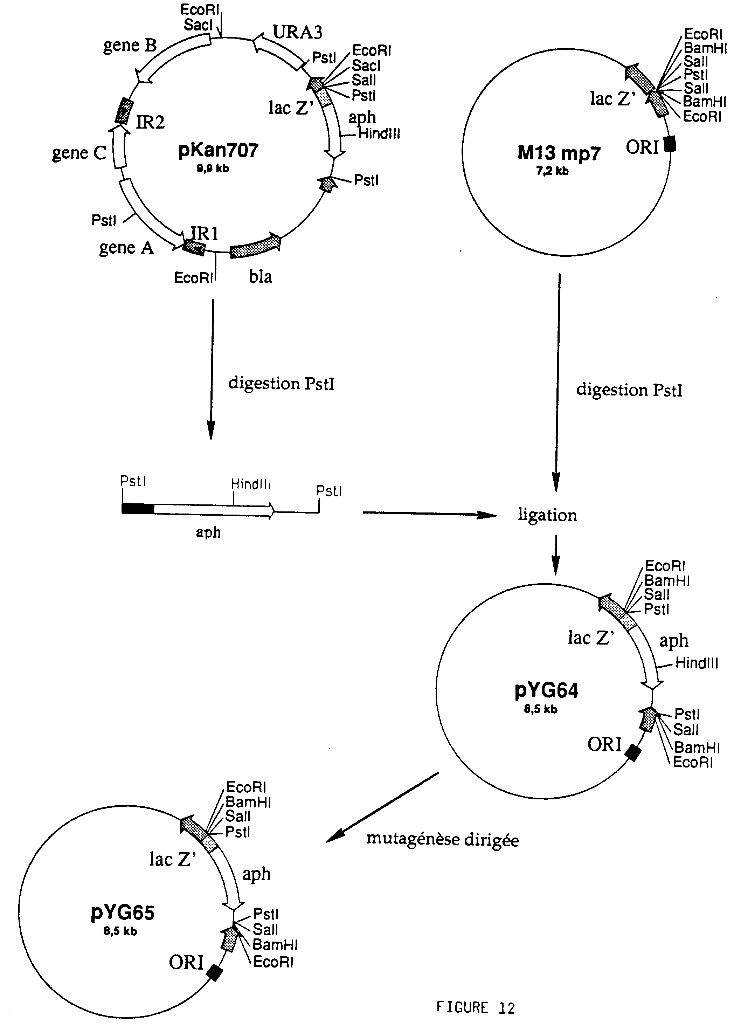

6.2.1. Construction du plasmide pYG140 (figures 12-14).

Un plasmide a été construit comprenant:
- un réplicon E.coli,
- le gène aph sous contrôle du promoteur k1 de la toxine killer de K.lactis (EP361911) dans lequel le site HindIII a été éliminé par mutagénèse dirigée, et
- le gène bla, conférant la résistance à l'ampicilline.

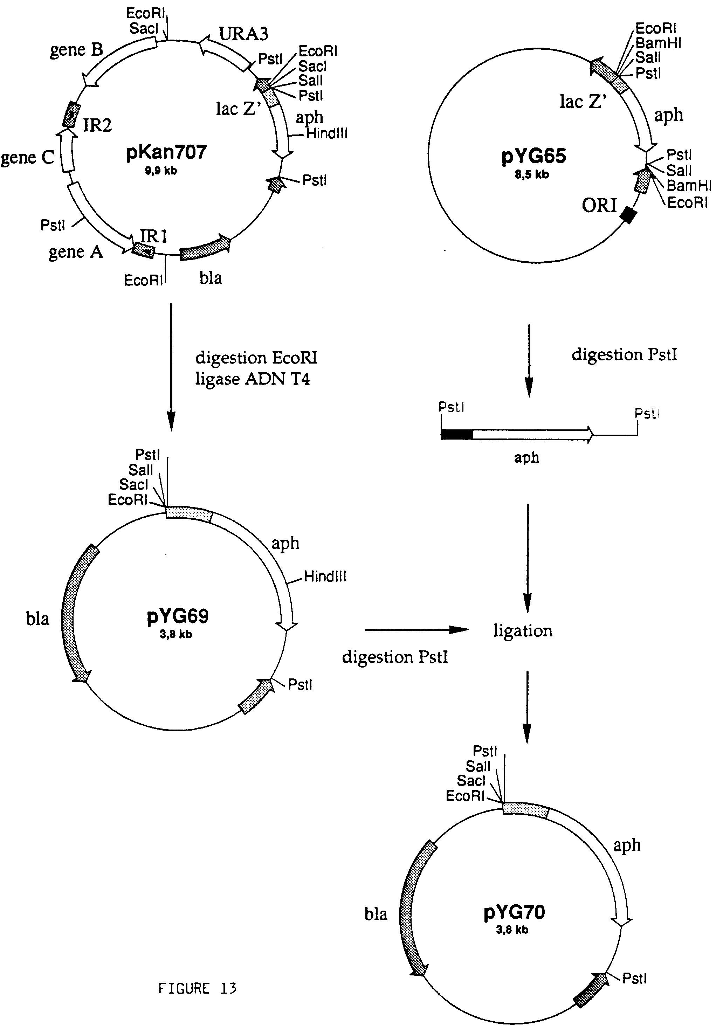

Le gène aph sous contrôle du promoteur k1 est isolé à partir du plasmide pKan707 (EP361991) sous forme d'un fragment PstI, qui est cloné dans le site équivalent du phage M13mp7. Le plasmide résultant est appelé pYG64 (figure 12). Le site HindIII présent dans ce gène a été détruit par mutagénèse dirigée selon la méthode décrite par Taylor et al. (Nucl. Acid. Res. 13 (1985) 8749). Le plasmide résultant est appelé pYG65. L'oligodéoxynucléotide ayant servi pour la mutagénèse a la séquence suivante: 5'-GAAATGCATAAGCTCTTGCCATTCTCACCG-3', et a permis de transformer le triplet CTT codant pour la leucine 185 en CTC. Pour construire le plasmide pYG70, la partie contenant le réplicon bactérien du vecteur pKan707 a été isolée par digestion avec l'enzyme EcoRI et recircularisation avec la T4 DNA ligase pour obtenir pYG69. Le fragment PstI présent dans ce dernier vecteur contenant le gène aph a ensuite été remplacé par le fragment équivalent muté provenant de pYG65. Le plasmide résultant est appelé pYG70 (figure 13).

Ce plasmide est ensuite digéré par EcoRI et religué en présence d'un adaptateur EcoRI-NarI-EcoRI contenant la séquence suivante : 5'-AATTCGGCGCCG-3'.

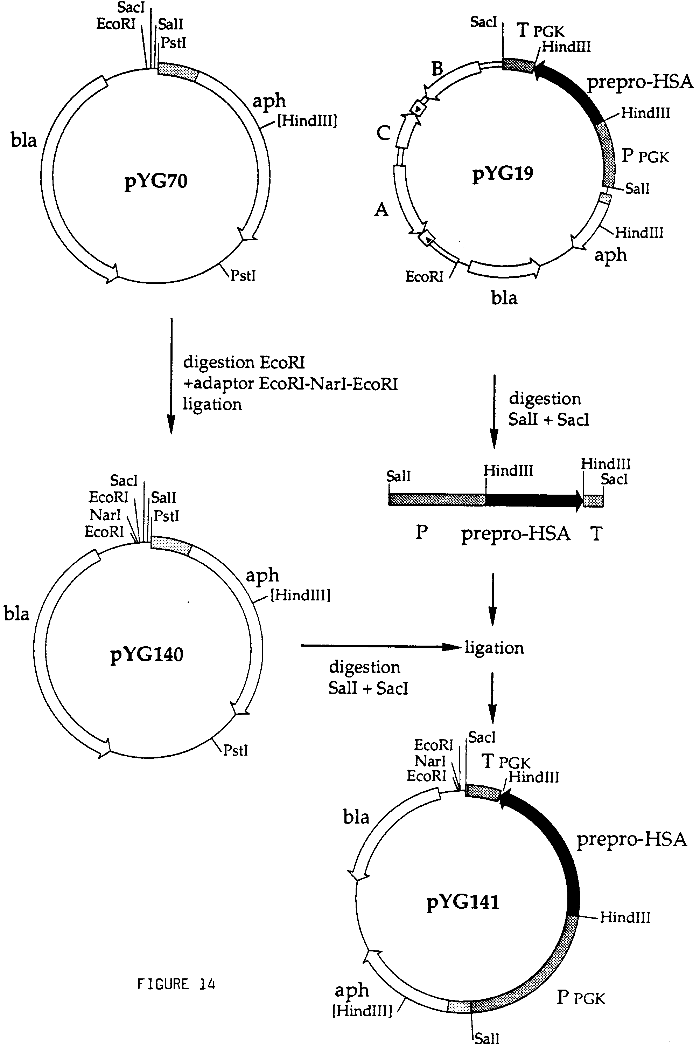

La plasmide obtenu est appelé pYG140 (figure 14).

6.2.2. Introduction d'une cassette d'expression de l'albumine (figure 14).

Le gène codant pour la préproSAH sous contrôle du promoteur et du terminateur du gène PGK de S.cerevisiae a été isolé sous forme d'un fragment SalI-SacI à partir du vecteur d'expression pYG19 (EP361991). Ce fragment a été introduit dans les sites correspondant du plasmide pYG140 pour générer le plasmide pYG141.

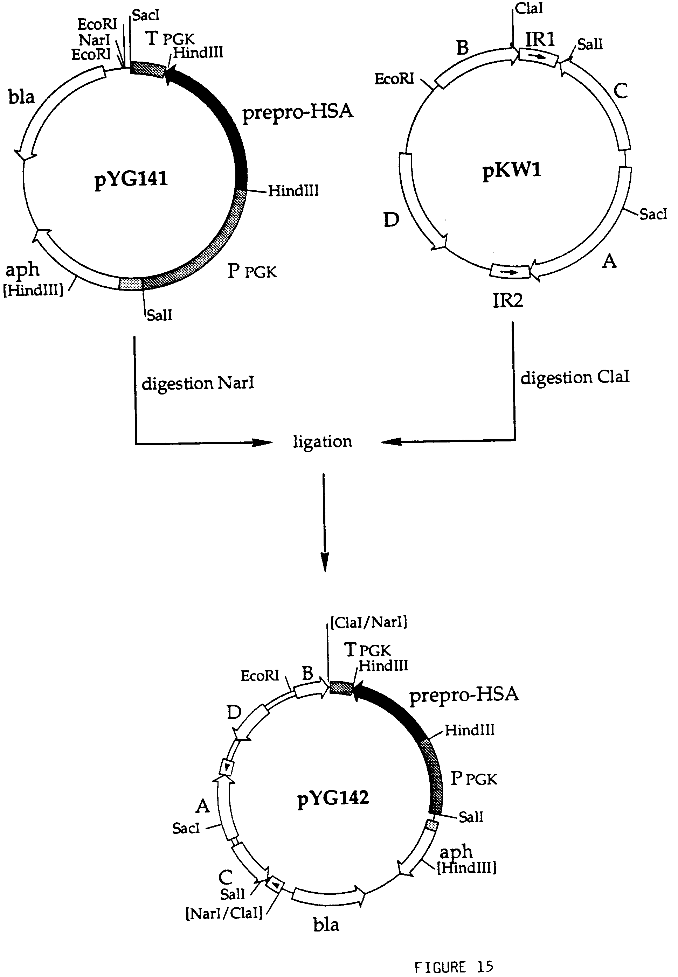

6.2.3. Construction du vecteur d'expression pYG142 (figure 15).

Les plasmides pYG141 et pKW1 sont digérés respectivement par les enzymes NarI et ClaI. Après ligation, 4 plasmides recombinants sont obtenus, en raison de l'existence des 2 formes A et B de pKW1, et de l'orientation de la partie pKW1 par rapport à la partie pYG141.

La figure 15 décrit la carte de restriction d'un de ces 4 plasmides : pYG142, contenant la forme B de pKW1.

Les autres plasmides sont appelés pYG143, pYG144 et pYG145.

Un échantillon de la souche K.lactis CBS 6430 a été déposé au CBS à Baarn (Pays-Bas) selon les conditions du Traité de Budapest le 4 juin 1991 sous le numéro CBS 290.91.

## TABLEAU 1

| VECTEURS | FRAGMENT DE PKW1 | VECTEUR NAVETTE | MARQUEUR |
|---|---|---|---|
| pBNA1 | BglII-NheI 2,4 Kb | YIp5 *BamHI-NheI | URA3 |
| pNEA2 | NheI-EcoRI 2,0 kb | YIp5 NheI-EcoRI | URA3 |
| pXXY2 | XhoI-XbaI 0,55 kb | YIp5 SalI-NheI | URA3 |
| pXXK3 | XhoI-XbaI 0,55 kb | pKan21 SalI-XbaI | Kan$^R$ |
| pBNB1/A3 | BglII-NheI 1,9 kb | YIp5 BamHI-NheI | URA3 |
| pNEB1 | NheI-EcoRI 2,5 kb | YIp5 NheI-EcoRI | URA3 |

TABLEAU 2

| VECTEURS | SITE DE LINEARISATION DE PKW1 | VECTEUR NAVETTE | MARQUEURS |
|---|---|---|---|
| pKWS14 | SalI | pKan21 *(SalI) | Kan$^R$ |
| pKWC11 | ClaI | pKan21 (AccI | Kan$^R$ |
| pKWS1 | SalI | YIp5 (SalI) | URA3 |

TABLEAU 3

Transformation de <u>Saccharomyces cerevisiae</u> par des vecteurs dérivés de pKW1.

| VECTEURS | Transformants Ura+ par µg d'ADN |
|---|---|
| pKWS1 | 4 400 |
| pBNA1 | 1 200 |
| pBNB1/A3 | 7 600 |
| pXXY2 | 4 000 |
| pSK1 | 4 500 |

EP 0 519 829 A1

TABLEAU 4

Transformation de <u>Kluyveromyces waltii</u> par des vecteurs dérivés de pKW1.

| VECTEURS | Support de réplication | Transformants G418-résistants par μg d'ADN | |
|---|---|---|---|
| | | pKW1$^+$ | pKW1$^-$ |
| pKWC11 | pKW1 total | 36 000 (98 %) | 8 000 (100 % |
| pXXK3 | XbaI-XhoI 540bp de pKW1 | 35 000 (49 %) | 10 000 (2,8 %) |
| pKWS14 | pKW1 total | 10 000 (92 %) | 8 000 (29 %) |

TABLEAU 5

Transformation de levures du genre <u>Kluyveromyces</u>.
avec le vecteur pKWC11

| Espèce | Souche | GC % | Fréquence de transformation par μg d'ADN | Stabilité des transformants (%) |
|---|---|---|---|---|
| K.waltii | CBS6430 | 45,6 | 2400 | 100 |
| K.thermotolerans | CBS6340 | 46,2 | 4000 | 25 |

**Revendications**

1 - Plasmide pKW1 isolé à partir de la souche <u>K.waltii</u> CBS6430, ou tout fragment ou dérivé de celui-ci.

2 - Fragment selon la revendication 1 caractérisé en ce qu'il s'agit d'un élément génétique.

3 - Plasmide caractérisé en ce qu'il comprend tout ou partie de la séquence présentée à la figure 3 ou d'un dérivé de celle-ci.

4 - Vecteur de clonage et/ou d'expression caractérisé en ce qu'il comprend tout ou partie du plasmide pKW1 de <u>K.waltii</u> CBS 6430 représenté à la figure 1 ou d'un dérivé de celui-ci.

5 - Vecteur selon la revendication 4 caractérisé en ce qu'il comprend un élément génétique au moins du plasmide pKW1.

6 - Vecteur selon la revendication 5 caractérisé en ce qu'il comprend l'origine de réplication du plasmide pKW1.

7 - Vecteur selon la revendication 4 caractérisé en ce qu'il comprend l'intégralité du plasmide pKW1.

8 - Vecteur selon la revendication 4 caractérisé en ce qu'il comprend tout ou partie de la séquence pré-

13

EP 0 519 829 A1

sentée à la figure 3 ou d'un dérivé de celle-ci.

**9 -** Vecteur selon l'une des revendications 4, 7 ou 8 caractérisé en ce que le plasmide pKW1 est linéarisé au niveau d'un site de restriction fonctionellement neutre.

**10 -** Vecteur selon la revendication 9 caractérisé en ce qu'il s'agit d'un site de restriction présent sur le plasmide pKW1, ou introduit artificiellement sur celui-ci.

**11 -** Vecteur selon la revendication 10 caractérisé en ce qu'il s'agit d'un site de restriction introduit artificiellement dans une région intergénique, et de préférence dans la région située entre les gènes B et D ou dans la région située entre le gène D et l'IR2.

**12 -** Vecteur selon la revendication 10 caractérisé en ce que le plasmide pKW1 est linéarisé au niveau des sites ClaI(1), PstI(4608) ou EcoRV(3072), les positions étant données par rapport à la figure 3.

**13 -** Vecteur selon l'une quelconque des revendications 4 à 12 caractérisé en ce qu'il contient en outre une séquence d'ADN hétérologue comprenant un gène de structure au moins sous le contrôle de signaux permettant son expression.

**14 -** Vecteur selon la revendication 13 caractérisé en ce que les signaux d'expression sont constitués par un ou plusieurs éléments choisis parmi les promoteurs, les terminateurs, et les signaux de sécrétion.

**15 -** Vecteur selon la revendication 14 caractérisé en ce que les promoteurs sont régulables.

**16 -** Vecteur selon la revendication 14 caractérisé en ce que les promoteurs dérivent de gènes de levure, et préférentiellement de gènes glycolytiques de levure.

**17 -** Vecteur selon la revendication 13 caractérisé en ce que le gène de structure code pour un polypeptide d'intérêt pharmaceutique ou agroalimentaire.

**18 -** Vecteur selon la revendication 13 caractérisé en ce que le gène de structure code pour une protéine hybride.

**19 -** Vecteur selon la revendication 13 caractérisé en ce que le ou les gènes de structure sont des gènes intervenant, au niveau génétique ou biochimique, dans la biosynthèse d'un métabolite.

**20 -** Vecteur selon la revendication 4 caractérisé en ce qu'il contient en outre un réplicon E.coli.

**21 -** Vecteur selon la revendication 4 caractérisé en ce qu'il contient en outre un marqueur de sélection au moins.

**22 -** Cellule recombinée contenant un vecteur selon l'une quelconque des revendications 4 à 21.

**23 -** Cellule selon la revendication 22 caractérisée en ce qu'il s'agit d'une levure.

**24 -** Cellule selon la revendication 23 caractérisée en ce qu'il s'agit d'une levure du genre Kluyveromyces ou Saccharomyces.

**25 -** Procédé de préparation d'un polypeptide caractérisé en ce que l'on cultive une cellule recombinée selon l'une des revendications 22 à 24 et on récupère le polypeptide produit.

**26 -** Procédé selon la revendication 25 caractérisé en ce que le polypeptide est choisi parmi les enzymes (telles que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur van Willebrand, la fibronectine, l'alpha-1 antitrypsine etc.), l'insuline et ses variants, les lymphokines [telles que les interleukines, les interférons, les facteurs de stimulation des colonies (G-CSF, GM-CSF, M-CSF...), le TNF, le TRF etc.], les facteurs de croissance (tels que l'hormone de croissance, l'érythropoïétine, le FGF, l'EGF, le PDGF, le TGF etc.), les apolipoprotéines, ou encore des polypeptides antigéniques pour la réalisation de vaccins (hépatite, cytomégalovirus, Eppstein-Barr, herpes etc. ).

**27 -** Procédé selon la revendication 26 caractérisé en ce que le polypeptide est l'albumine humaine ou un de ses variants ou précurseurs.

**28 -** Utilisation d'une cellule recombinée selon l'une des revendications 22 à 24 comme catalyseur dans une réaction de bioconversion.

14

FIGURE 1

Figure 2

```
          |  10      |  20      |  30      |  40      |  50      |  60
   1 ATCGATTGCC AGCAACAGGG TTTTGCGCGT ATGTCTCTTG TGGATACTAT GTAAACAAAA 60
  61 CAATCAATGT ACAAAGAGCA CAGCGGCCGG CAGGTGGGAG GACCCTCTGA TGAGCCGGGG 120
 121 ATATGGCGCT CCCGCGCGTC TAATATCCGG ATTGGACTGG AGGAGGACCA AGGTTTCCTC 180
 181 GAGGTCATGT GCCGCATCAT GAGGGACGAA AAATGGTAAG GAATAGACCA TTCCTTACCA 240
 241 TTTTTCGTCG CCCGTGAGAT TTTCCCATTT CCCGTTCCTT ACCATTTTTC GTTCCGCACG 300
 301 ACCCTATCAC ATTGTATATT GAAATCTACT TCCCTTCATA CCGCTTGGCC ATCGCGTAGG 360
 361 AATGAACATA TGTAGCCCCT CTAGTTCCTC ATGTTGTTCT CAATCGACCC TTCCATCGTG 420
 421 AAGGGATCTC CTCTTGAGGC AATGTCGACG TTCGTACCCT CTTCCTACGG GTCACGGGTT 480
 481 CGTCTGTAAC ACTTCTTCTT GGTCTCCCTC GGCCTCTTTT ACTTGGCTGT GGCCTGGTAT 540
 541 TTTTGAGTTC ATCGAATAGG CGCGGGTTTT CTTTTACTTG AGGCCAGTCT TGAATCTCTG 600
 601 AACCGAGAGA GGTTAGAGAA GTTATTCTTT TTAGATCATT TAACATGTCC TTGATGTCTT 660
 661 CTGGCGTTGT TTCGAAGCAC ATCAGTCCCC TTGAGCGATA TCTGTCGTAA TTTGCCAGTA 720
 721 TTACGTTGTG CGCTTCTTCC ATGTTTTCAG GGGCCACATG CTTTAATGCC TGCACTAAAA 780
 781 CACTTCTGAC AAGTGACCAA TCTGCGAATG CAGGCTCGTT CGCCAATATC AATCTCTGTA 840
 841 GAGGAACACC ACGATAGCTT GTTGTGCCCT TGCAGATAGT GTTCAAGAAT AGACTGAGGT 900
 901 AGCCACGTAC GTCGAGCTTT ACAGACCTGG TGCCAACGCC AGAAAGAAGG CGAAGGGGT 960
 961 CCTCTTCAAT CGGAAAACGG GTCAGGCTAT CCACACGGTA CTCTCTATAT GTACTACGAG 1020
1021 TCATCGTCGT GTACTTTGCG AAGTGCAGGC CGTGGCGATT ATAGTCAATC TGGAAAGAAT 1080
1081 CCTGCTCATG TGGCTCTTTC TCCCCATTAT CCTCTTCAGA GTTCAGCGAG CCAGATGGCA 1140
1141 GCTCGGCTGG CTCTCGCAGG TACGTGGGAT CGGGCTCACC TCGATCCACC GTCATCTCAT 1200
1201 CAAGATTGCA CATAATCTGC ATCAGTCTCA ACGTCATATC GACCGCGGAA AAGGCGTCCG 1260
1261 AGTAGGAAAC ATGCAGTTTG GAAAGGTCTA ACAGCTTGAG GCAGTGCATC TTGGCCTCTC 1320
1321 TCTCTGTCAG TCTTTCTCTG TTTTGACCAA TTTTTCTCAA GTGTATTACA TTTGTTTCTT 1380
1381 GGATTGCAAA AATGTTGCGA GTACTGCGTG TTCTCGTTCA AAAACAGCGT TCGCTGAAAA 1440
1441 TTTAGGAGTT CAGATGCAAC GCGTAGTGCA AATGGAGGAT TCAAGTTGCA GTAACAATAA 1500
1501 CATGGAACAC CAAGGATCAG TGTTCGAGGA GCTTATCTCC AAAAATCTTA TGAGCCTGAT 1560
1561 GGAAGAATTG ATGTCTATGC TCACTAATGA GAAGGAGTTC CAACGTGAAA GGTTCGCGTC 1620
1621 TCTCCTAGCC TACATGATAA TGGCTACTGG TGAATTGGAA GAGAAAAAGC TCAGTACATT 1680
1681 TACCAAGTAT TCCCGACGGA TCAGGCAGAC GGTAGAGTTC GACAGCAACA ACCAAATCGT 1740
1741 AAGATTTGAG TACCATTTGA AGAATCCCAC AGAGCTCAAG GAGACGCTGG ACAAGGCCTT 1800
1801 TAAACCTGTC GTGTTTGAAA TCAAGTCCAA AAAGAAGGTT GTCTCCATGC TGGAGCTAGC 1860
1861 TGCGAAGCTC GACAAAAGGG GATCAGATTC AGCGGGTGGT ACGGTAGCTA GTGAGGTCTC 1920
1921 GAAGCTTGTG CGGGAGGAAG AAATTTGGCT TCTTCTCGTG AACGTGAAGA ATACTATCCA 1980
1981 GGAAAAGGTG CGCAAATCAT CGCTAAGAGC GGAATTGACG TATATTTTGA CAGCCTCATT 2040
2041 CTTCAATTGT TGCAGACATA GCGATCTCAG GAACGCAGAC CCCGCAACAT TTGAGCTGGT 2100
2101 GCCAAATAAG TATGTGGGCC ACGTTGTCCG GGTTTTGGTG TGCGAGACCA AGACCCGAAA 2160
2161 GCCGCGGTTC ATATACTTTT TCCCTGTCAA TACGGCCGCG GATCCTCTAG TAGCGCTTCA 2220
2221 TGATTTGTTC TCGAGCACGT TTCCTTCCAA AAAGAGTCGG ACGTCCGAAA GAAAGCAGGA 2280
```

Figure 3 (a)

17

```
2281  ACAGGAATGG  CAGATCGTTC  GCGACGCATC  AATCAACAAC  TATGACCGGT  TTGTTGGTAA  2340
2341  GCACGCTACG  GAATCTGTCT  TTGCCATCTT  GCATGGTCCC  AAATCACACT  TGGGCCGGCA  2400
2401  CTTGATGAGT  TCCTACTTGG  CGTATACCCA  CCATGGGGAA  TGGGTCTCAC  CATATGGGAA  2460
2461  TTGGTCAGCT  GGGAAAGGAA  CCATTGAAAG  CAGCGTGGCA  AGGGCCAAGT  ACGCACATGT  2520
2521  TCAAGCCGAG  ATCCCAAGCG  ATCTTTTCGC  CTTTCTGTCT  CAGTACTATC  AGGAATCAAA  2580
2581  ATCGGGCGAT  TTCGAGCTTA  ACGACACCAG  CAAAGACCCA  ACAAAGCTGG  TACGGCACTC  2640
2641  GGCTAGTCAA  CTGGAAATCA  ATCGAACCTA  TGGTCCATGG  AGTAGATTGG  TTAACAAGGA  2700
2701  TGTTTTAGGC  TTTGTTCATT  CCTACGCGAT  GGCCAAGCGG  TATGAAGGGA  AGTAGATTTC  2760
2761  AATATACAAT  GTGATAGGGT  CGTGCGGAAC  GAAAAATGGT  AAGGAACGGG  AAATGGGAAA  2820
2821  ATCTCACGGG  CGACGAAAAA  TGGTAAGGAA  TGGTCTATTC  CTTACCATTT  TTCGTCCCTC  2880
2881  ATGATGCGGC  ACATGACCTC  GAGGAAACCT  TGGTCCTCCT  CCAGTCCAAT  CCGGATATTA  2940
2941  GACGCGCGGG  AGCAGCCTAT  CCCCGGCTCA  TCAGAGGGTC  CTCCCACCTG  CCGGCCGCTG  3000
3001  TGCTCTTTGT  ACATTGATTG  TTTTGTTTTA  GTATTACCTG  ACAATCATTT  TATATTTTGT  3060
3061  CAGTATTTCT  TGATATCTGG  CCCAAATGAA  AGTACAAATA  CAAGTACAAG  TACAACACCT  3120
3121  ATTCATTGTT  ACCGTATCTC  TATACTATTA  TCCTTATTTC  CTGCCTAATT  AACTACTTTC  3180
3181  TACCGGTGCG  TTCTTAAAGC  TGAGTAGGCA  CTTCAGCCCG  AAGTGAATAT  CGCTGGCCTC  3240
3241  ATTATCTGTT  TGTTGTATGG  CCAATGAAAC  TCCAACCGAT  TTGATCCACT  TGCAAGGACC  3300
3301  GTAGGATAGA  CTTATTTTTA  CCATCCCATT  ATCTGTGCAG  TGGACACCTC  TGTTTATCTT  3360
3361  TACCCGCCCT  TCAGAGAACA  TAATCTGTCT  TACAAAAGAG  TAGACCTCAT  TTTTGGCGAG  3420
3421  AAGCCTCTTC  TGTTTGACCT  CATCTAGAAG  CTTTTGGGCT  CTCCCAGCGC  AATCAGATAG  3480
3481  TCTTGTGAGA  GTGTCCTCTT  CGTCACCATG  CTCATCAGAA  GGAGCTGGTT  GCGTTCCAAT  3540
3541  TGAGAAAGCT  CGTCCCAAAA  GGCTGCATGG  TCTATAGGTG  GTCCCGAACC  CGAGGATTCA  3600
3601  CCTTGAGACT  CATTGTCCAC  AGTAGGCTCC  TGATCCTCTG  CAGCAGCCGC  TTGCTGAATG  3660
3661  GATTCTGCTC  TTGACATTAC  CATTTCGAGA  AGCCATAGTC  TTCGGATGGT  CTAGCAGAT  3720
3721  CTTTCAACAT  CCAAACTGGA  ACCGAACCAT  TGCCGACAAC  TTATTCTAAC  CTCAAGCTTT  3780
3781  GAGAGGTTCT  CATACTTGGA  TTGTGCAGCC  TCAAGATCCG  TAAGTTCGGT  GAAGAAATCT  3840
3841  AAAGCTTCGC  GTTTTGGACC  TAACCGAATG  ATGATTGGGT  GCCGTTTTCG  TGGATCCAGA  3900
3901  AATGGTGTAA  GCTCCTCTAT  ACTATCATTC  GTGGGACACT  CTATCCCTTC  CCAATATGC  3960
3961  TTCTTTACAA  TAGAAGGTAG  CTGCGTATAC  TTGTTCCGAA  CAACAAAGAT  GTGACTCTCT  4020
4021  AGCCGCTTTA  CTATTGTGTT  CAACACAACG  TAGGGTTTTG  GCCACTCAAA  TGTTAATTCT  4080
4081  GATCGAGCTG  CTGATGCTGT  GTTCTTGTTG  ATCATGTATG  AATAGTAGAA  CAATGCCAAT  4140
4141  TGAGGATGCT  CACTGTATTC  CTTAGGCAGT  TTGGTGCTGC  CATAAGGTGA  CACCAGCTCC  4200
4201  TTTAGCTGTG  AACATAGAG  TACATCAACA  GTTCCTACTA  GACAACATTC  CTTGAGACAA  4260
4261  TTATGATAGC  CATCATGGTC  GGTTATCTTA  GGTATTTTTT  GAGCTTCACT  CATCTTCGAA  4320
4321  GCAACCGTGA  TAGATTCGAT  TGAAGTTCAA  ATTCTTATCT  AGATGGTGTA  TTTGTTTATC  4380
4381  ATAATTTACA  ATACAGTCTG  TTTTAATTTG  CTCGAAGTTG  CAGTGAAAGA  TGTAAAAAGG  4440
4441  GGCTTCATTT  TGCACTACAG  ACTTACCCTG  ATGTAAAAAA  TTTTCATCAT  AAAAGCAATT  4500
4501  TCTCGTATAC  AAAATGCAGT  TTGTTTCGTA  TACAGCAAAG  CATAGAATAC  ACTGTACACT  4560
4561  GCATTTTTAC  TACAAGAAAA  GTTTTTTTTG  CTGCTGTGAC  TGGAATGCTG  CAGCAGTACC  4620
```

## Figure 3 (b)

```
4621 TAGATAGAAA AATGGCATAA AACTGAAATT TTATAGTCAT TTTTCGTGTC TTTCATTCAA 4680
4681 TTTTTTCTCG CAAAAGTTTT CTACAAAAGC AGTCAAAATT GCAATAAGTA TACACTAATT 4740
4741 ACAAGGCGTC TGAGCGCGTG ACTTGAGCGC GTGACGTAAT CGCGAGCTAC GAAAGTTGTT 4800
4801 TGGGCCTCAG ACATCGGATC GACAGAAGAG GTAAGAATAT TGGGAAAGTA CATTCAATTA 4860
4861 CCACAACAAT CGAGAGATTA GTGGAATTCA GTCATTAATG AAAGGTAGGG TAGTCCGCCT 4920
4921 ACTCTTAGTT CTACATTCAG AATACGCATG CAGTCCTCAA GTTCTGATGA AGACGACCTA 4980
4981 ATTGACCCTA TAATTCATCC CAAATCGTTC TATAGGGCGG CTAACGAGAT ACCGAGAGAC 5040
5041 TTTTTGGTTG CGATCCCCAT CAGCGCCTAT GTTTTTAGCG TATTTGCTAA ATCAGTACGA 5100
5101 GATGACTTAC AGGGGCATTT AACGGCGCGA GATATGGCAT TAGCTTATCG TGAACGGCAG 5160
5161 TACTTTCACA GACGCTGGGA GACACGAAAC GACCAGCTTG AGATCCCAGA CTGGTCTGAG 5220
5221 ATCCCAGAAT GGTCTCTCGG GTTACTGGAT CGCCCTCCTT GTATCACTGT GGATCTAGCA 5280
5281 AGAGAACTGC GCGAACTATC TCAAAAATGG ATCGGAGCAT TCGATCTGGG ATCGAAGATG 5340
5341 TCTGGCAGGC TTCTTCTACA GCTTCTGTAC ACCCAGCTGT CATGCCCAAA TGAGGCTGTT 5400
5401 TTCAATAAGC TTTACTGCCT CGTCAAACTA CTAAACAAGG ACGTAAATCG TGCGGACCGA 5460
5461 GCCCTTATGG ACTCTGTATT GAGACCACTT TTTGTCGAGA ATCCATACAT GGGTGAACTA 5520
5521 GATGAAGAAA TACTTGATAA GATATGGTCC AATTTGACTG AAATGAGAAG TCAAGAGTGG 5580
5581 AAACGTATAG CGGAAGCGTT GTCAGGCGAG AATAATGAC                        5619
```

Figure 3 (c)

FIGURE 4

FIGURE 5

Figure 6

Figure 7

Figure 8

FIGURE 9

FIGURE 10

26

FIGURE 11

FIGURE 12

FIGURE 13

FIGURE 14

30

FIGURE 15

European Patent
Office

**EUROPEAN SEARCH REPORT**

Application Number

EP    92 40 1711

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 635 115 (RHONE-POULENC SANTE) 6 February 1990 --- | | C12N15/81 |
| P,X | JOURNAL OF GENERAL MICROBIOLOGY vol. 138, no. 2, February 1992, COLCHESTER, UK pages 337 - 345 X.J. CHEN ET AL. 'Characterization of a circular plasmid from the yeast Kluyveromyces waltii' --- | 1-28 | |
| A | EP-A-0 301 670 (GIST-BROCADES N.V.) 1 February 1989 --- | | |
| A | CURRENT GENETICS vol. 19, no. 3, 1991, BERLIN, GERMANY pages 163 - 167 C. WILSON AND H. FUKUHARA 'Distribution of mitochondrial r1-type introns and the associated open reading frame in the yeast genus Kluyveromyces' ----- | | |

TECHNICAL FIELDS
SEARCHED (Int. Cl.5)

C12N

The present search report has been drawn up for all claims

| Place of search | Date of completion of the search | Examiner |
|---|---|---|
| THE HAGUE | 09 OCTOBER 1992 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document